(19) Europäisches Patentamt
European Patent Office
Office européen des brevets

(11) **EP 4 396 769 B1**

(12) **EUROPEAN PATENT SPECIFICATION**

(45) Date of publication and mention
of the grant of the patent:
**20.11.2024   Bulletin 2024/47**

(21) Application number: **22753611.7**

(22) Date of filing: **14.07.2022**

(51) International Patent Classification (IPC):
**G06T 7/00** *(2017.01)*      **G06T 7/11** *(2017.01)*

(52) Cooperative Patent Classification (CPC):
**G06T 7/0012; G06T 7/11;** G06T 2207/10081;
G06T 2207/20084; G06T 2207/30004;
G06T 2207/30096

(86) International application number:
**PCT/EP2022/069725**

(87) International publication number:
**WO 2023/030734 (09.03.2023 Gazette 2023/10)**

(54) **METHOD AND COMPUTER PROGRAM FOR THE AUTOMATIC CLASSIFICATION OF PANCREATIC CYSTS**

VERFAHREN UND COMPUTERPROGRAMM ZUR AUTOMATISCHEN KLASSIFIKATION VON PANKREASZYSTEN

PROCÉDÉ ET PROGRAMME INFORMATIQUE POUR LA CLASSIFICATION AUTOMATIQUE DES KYSTES PANCRÉATIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.08.2021   EP 21382787**

(43) Date of publication of application:
**10.07.2024   Bulletin 2024/28**

(73) Proprietor: **SYCAI TECHNOLOGIES, SL
08029 Barcelona (ES)**

(72) Inventors:
- **RIERA MARÍN, Meritxell**
  08025 Barcelona (ES)
- **RODRÍGUEZ COMAS, Júlia**
  08015 Barcelona (ES)
- **TOLEDANO SÁEZ, Sara**
  08029 Barcelona (ES)
- **GARCÍA LÓPEZ, Javier**
  08029 Barcelona (ES)

(74) Representative: **Torner, Juncosa I Associats, SL
C / Pau Claris, 108, 1r 1a
08009 Barcelona (ES)**

(56) References cited:
**WO-A1-2020/240455      CN-A- 108 898 152**

- **DMITRIEV KONSTANTIN ET AL: "Classification of Pancreatic Cysts in Computed Tomography Images Using a Random Forest and Convolutional Neural Network Ensemble", 4 September 2017, COMPUTER VISION - ECCV 2020 : 16TH EUROPEAN CONFERENCE, GLASGOW, UK, AUGUST 23-28, 2020 : PROCEEDINGS; PART OF THE LECTURE NOTES IN COMPUTER SCIENCE ; ISSN 0302-9743; [LECTURE NOTES IN COMPUTER SCIENCE; LECT.NOTES COMPUTER], SPRINGER INTERNATIONAL PU, ISBN: 978-3-030-58594-5, XP047527848**
- **ATTIYEH MARC A ET AL: "CT radiomics associations with genotype and stromal content in pancreatic ductal adenocarcinoma", ABDOMINAL RADIOLOGY, SPRINGER US, NEW YORK, vol. 44, no. 9, 26 June 2019 (2019-06-26), pages 3148 - 3157, XP036859230, ISSN: 2366-004X, [retrieved on 20190626], DOI: 10.1007/S00261-019-02112-1**

**Description**

TECHNICAL FIELD

The present invention refers to a method and computer program products for the automatic classification of pancreatic cysts. The invention allows obtaining the exact position and volume of a pancreatic cyst, particularly on computed tomography (CT) images, with a diameter greater than 5mm in its section.

BACKGROUND OF THE INVENTION

Detection of pancreatic cystic lesions is crucial since this kind of lesions can evolve into pancreatic cancer. In practice, the types of pancreatic cysts are defined in the different clinical guidelines. Generally, these guidelines define the following nomenclature for each type of cyst, which also statistically determines its probability of leading to pancreatic cancer:

- Intraductal papillary mucinous neoplasia (IPMN) is a type of neoplastic cyst that normally affects the main pancreatic duct or Wirsum. In 40% of the cases it is multicystic without this having a proven impact on the probability of malignancy. IPMN typically has a pleomorphic cluster shape.

- Mucinous cystic neoplasm (MCN) is a type of cyst that is typically found on the body or tail of the pancreas. This type of cyst does not normally have access to the main pancreatic duct.

- Serous cystadenoma (SCA) is a benign type of pancreatic cyst. SCA has a low internal viscosity and consist of a typically lobular morphology.

- Pseudocysts (PCYST) are a type of non-malignant and non-neoplastic cysts with a round or oval shape.

Pancreatic cysts and their classification are often difficult to appreciate through visual analysis. Given its importance in the health of the patient, an early classification as accurately as possible is very important in order to be able to perform an early prediction of the probability of it ending up being pancreatic cancer.

There are known some patents and patent applications in this field.

EP3646240-A1 discloses a system, method, and computer-accessible medium for using medical imaging data to screen for a cystic lesion(s). The method can include, for example, receiving first imaging information for an organ(s) of a one patient(s), generating second imaging information by performing a segmentation operation on the first imaging information to identify a plurality of tissue types, including a tissue type(s) indicative of the cystic lesion(s), identifying the cystic lesion(s) in the second imaging information, and applying a first classifier and a second classifier to the cystic lesion(s) to classify the cystic lesion(s) into one or more of a plurality of cystic lesion types. The first classifier can be a Random Forest classifier and the second classifier can be a convolutional neural network classifier. The convolutional neural network can include at least 6 convolutional layers, where the at least 6 convolutional layers can include a max-pooling layer(s), a dropout layer(s), and fully-connected layer(s).

WO2021096991-A1 discloses methods, systems, and apparatuses, including computer programs for detecting pancreatic neoplasms. A method includes providing an image as an input to a first model, obtaining first output data generated by the first model based on the first model's processing of the image, the first output data representing a portion of the image that depicts a pancreas, providing the first output data as an input to a second model, obtaining second output data generated by the second model based on the second model's processing of the second input data, the second output indicating whether the depicted pancreas is normal or abnormal, providing the first output data and the second output data as an input to a third model, and obtaining third output data generated by the third model, the third output data including data indicating that the pancreas is normal or data indicating a likely location of a pancreatic neoplasm.

US20210012505-A1 relates to methods, systems, apparatus, and computer programs, for processing images through multiple neural networks that are trained to detect a pancreatic ductal adenocarcinoma. In one aspect, a method includes actions of obtaining a first image that depicts a first volume of voxels, performing coarse segmentation of the first image using a first neural network trained (i) to process images having the first volume of voxels and (ii) to produce first output data, determining a region of interest of the first image based on the coarse segmentation, performing multi-stage fine segmentation on a plurality of other images that are each based on the region of interest of the first image to generate output data for each stage of the multi-stage fine segmentation, and determining based on the first output data and the output data of each stage of the multi-stage fine segmentation, whether the first image depicts a tumor.

EP1421544-A1 provides a method of detecting and analyzing abnormalities, like lung nodules, in thoracic computer tomography (CT) images using digital image processing techniques and adaptive computing methods. The techniques include an automatic detection process to detect candidate abnormalities, an image matching process to match CT slices from two different CT scans, and a measurement process that determines parameters of the candidate abnormalities. Final results and processed CT images are displayed on a user interface.

WO2020240455-A1 describes a computer based method to characterize a mass of an organ such as a cyst and to provide a class that indicates the presence of a serum and solid in the cyst and a subclass that indicates the presence of one or more serous or solid loculi in the cyst. The method includes the steps of: (a) obtaining at least an ultrasound image of an examined anatomic entity including the anatomical mass; (b) using a first algorithm based on a neural network that processes the image to generate the characterization as a cyst and/or the class; (c) use a second algorithm of computational graphics for: identifying internal regions of the image, classifying the internal regions as serous or solid on the basis of at least one quantitative morphological parameter of the internal region, and counting the number of fluid regions; (d) providing a classification comprising the class and subclass based on the combination of outputs of the first and second algorithms.

CN108898152-B discloses a pancreatic cystic tumor image classification method based on multi-channel multiple classifiers. The method comprises: 1) performing window width and window level adjustment on an original image, and performing Canny edge detection and gradient amplitude calculation to enhance edge features; 2) adopting a ResNet to perform end-to-end training on a multi-channel graph, using an output of a pool 5 layer as extracted features, using a Bayesian classifier and a KNN classifier to perform classification, and obtaining the classification probabilities; and 3) using a random forest classifier to classify the obtained 3 different probabilities to get a final result, wherein a random forest is composed of multiple decision trees. The pancreatic cystic tumor image classification method basedon multi-channel multiple classifiers can automatically perform edge enhancement, and can improve classification accuracy.

Moreover, Dmitriev et al. "Classification of Pancreatic Cysts in Computed Tomography Images Using a Random Forest and Convolutional Neural Network Ensemble" describes an automatic classification algorithm that classifies the four most common types of pancreatic cysts using computed tomography images. This proposed approach utilizes the general demographic information about a patient as well as the imaging appearance of the cyst. It is based on a Bayesian combination of the random forest classifier, which learns subclass-specific demographic, intensity, and shape features, and a new convolutional neural network that relies on the fine texture information.

Attiyeh et al. "CT radiomics associations with genotype and stromal content in pancreatic ductal adenocarcinoma" investigates the relationship between CT imaging phenotypes and genetic and biological characteristics in pancreatic ductal adenocarcinoma (PDAC). The study demonstrates that in PDAC SMAD4 status and tumor stromal content can be predicted using radiomic analysis of preoperative CT imaging. These data show an association between resectable PDAC imaging features and underlying tumor biology and their potential for future precision medicine.

## DESCRIPTION OF THE INVENTION

An object of the present invention is to provide an automatic segmentation method capable of locating and characterizing pancreatic cysts on medical images, particularly CT images.

This object is fulfilled by a method with the characteristics of claim 1 and by a computer program product with the features of claim 11.

The invention finds special application in the field of medicine, specifically for assistance in the diagnosis and premature classification of pancreatic cysts and the study of their temporal evolution.

Embodiments of the present invention provide, according to a first aspect, a computer implemented method for the automatic classification of pancreatic cysts using CT images, the method comprising: accessing a set of CT images of a patient, each image of the set of CT images representing a different slice; performing a filtering operation on the set of CT images, and defining a Region Of Interest (ROI) candidate to contain a pancreatic cyst within the filtered set of CT images; performing a segmentation operation of the defined ROI using a neural network, obtaining a segmented image as a result, the segmented image representing values of a first value for the pixels occupied by a pancreatic cyst and values of at least a second value for the pixels not occupied by a pancreatic cyst; performing a morphological analysis of the pancreatic cyst by means of using an image processing algorithm; and classifying the pancreatic cyst based on the morphological analysis performed in the previous step and demographic data of the patient, the demographic data including the gender and age of the patient.

Particularly, according to invention, the characterization of the pancreatic cyst is obtained through three main indicators: shape factor (eccentricity, convexity), relative position in the pancreas, and size.

Therefore, the morphological analysis particularly comprises computing a value of eccentricity and of convexity of the pancreatic cyst by means of the image processing algorithm processing the pixels with the first value.

In an embodiment, the value of eccentricity of the pancreatic cyst is computed by calculating the major and minor axes from a middle section of the pancreatic cyst. The value of the convexity can be calculated as a proportion between the volume of the pancreatic cyst and the volume of the respective convex hull.

The morphological analysis can further comprise the calculation of a value of a relative position of the pancreatic cyst by the image processing algorithm dividing the pixels with the second value in three thirds in a 3D space and checking a 3D position of the pancreatic cyst within the divided three thirds.

Moreover, in some embodiments, the morphological analysis of the pancreatic cyst can further include computing one or more of the following features of the pancreatic cysts: if the cyst is multicystic, if the cyst has calcifications, and if so the location thereof, if the cyst has air balls, and if so the location thereof, if there are scars inside the cyst, if the cyst is flat, lobulated, circular or ovoid, an aspect of the cyst and/or if the cyst accesses a pancreatic duct or not.

The classifying step can comprise classifying the pancreatic cyst in four different groups, namely: Intraductal papillary mucinous neoplasia (IPMN); Mucinous cystic neoplasms (MCN); Serous cystadenoma (SCA); and Pseudocysts (PCYST).

In some embodiments, the segmented image represents three different values: the first value for the pixels occupied by the pancreatic cyst, the second value for the pixels occupied by the pancreas and a third value for the rest of the pixels. The objective of the segmentation is to simplify and/or change the representation of a filtered image in another more significant and easier to analyze, being used both to locate objects and to find the limits of these within any image.

In an embodiment, the filtering operation comprises a median filter, for example, a 3 x 3 filter window. The main objectives pursued with the application of the median filter are smoothing the image and reducing the amount of intensity variations between neighboring pixels.

In an embodiment, the neural network comprises a semantic segmentation algorithm. Particularly, the neural network is based on deep learning, U-Net.

In some embodiments, the demographic data of the patient can also include an ethnic group, a medical history, and/or a medication intake of the patient.

Other embodiments of the invention that are disclosed herein also include software programs to perform the method embodiment, steps and operations summarized above and disclosed in detail below. More particularly, a computer program product is one embodiment that has a computer-readable medium, including computer program instructions encoded thereon that when executed on at least one processor in a computer system causes the processor to perform the operations indicated herein as embodiments of the invention.

Present invention solves problems that were not solved in the current state of the art, taking into account the following aspects: 1. It allows to have the location in CT image of a pancreatic cyst with high precision; 2. Its application makes possible to obtain a three-dimensional characterization of the pancreatic cyst that determines its probable automatic classification among one of the types defined in the clinical guidelines.

## BRIEF DESCRIPTION OF THE FIGURES

The previous and other advantages and features will be more fully understood from the following detailed description of embodiments, with reference to the attached figures, which must be considered in an illustrative and non-limiting manner, in which:

FIG. 1 is a flowchart illustrating an embodiment of the proposed method.

FIG. 2 is another flowchart illustrating the characterization and classification of a detected pancreatic cyst, according to an embodiment.

## DETAILED DESCRIPTION OF PREFERRED EMBODIMENTS

Various embodiments discussed herein can construct and/or employ methods to automatically characterize and classify pancreatic cysts, particularly using computed tomography (CT) images.

Present invention allows accurate and reliable measurements of the location and type of pancreatic cyst in abdominal imaging studies, more specifically from their representation on the axial axis so that they can be used as a measure of the probability that this cyst leads to pancreatic cancer.

For the acquisition of the images, any image obtained on a CT equipment for clinical use can be used.

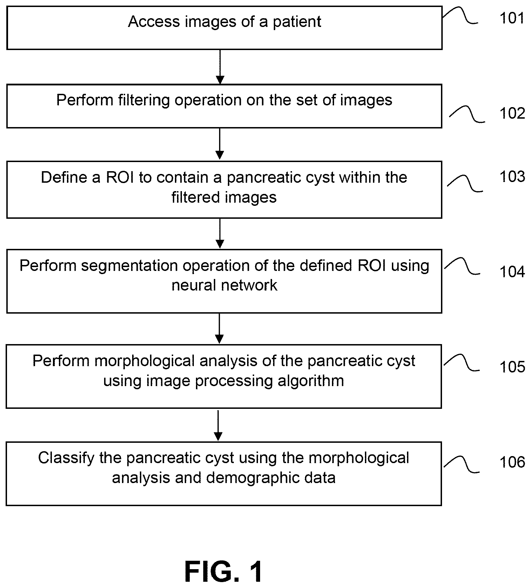

With reference to FIG.1 therein an embodiment of the proposed method is shown. According to this embodiment, at step 101, a set of images of a patient are accessed (or received) by a computer including one or more processors and at least one memory.

In some embodiments, the computer can be a server computer, a PC, a tablet or even a smartphone. The computer can include a single or several hardware modules, or a combination of one or more software modules and one or more hardware modules.

In some embodiments, the set of images is previously organized and stored in directories based on the DICOM information of each sequence of CT images before being accessed (or received) by the computer.

At step 102, a filtering operation on the set of images is performed, for instance by applying a median filter; hence noise from the acquired images is reduced. It should be noted that the properties of the filter will depend on the spatial resolution of the images. At step 103, a region of interest (ROI) candidate to contain a pancreatic cyst is defined within the filtered set of images.

Once the ROI is defined, at step 104, a segmentation operation is further performed by means of executing a neural network such as, but not limited to, a U-Net.

The U-Net algorithm consists of two branches known as "encoder" and "decoder". The first is an achievement of 8 convolution operations through 3x3 dimension filters and four max-pooling operations interleaved each one with a sequence of two convolutions. The goal of this first branch is to reduce the size of the input image to a resolution of 32x32 pixels. During this reduction, the aim is to learn the value of the convolution kernels and their weights that are best extracted from the context of the image (areas where the cyst is likely to be found). The second branch of this algorithm performs a rescaling from 32x32 pixels to the initial resolution and is made up of a number and type analogous to the first branch. This second part of the architecture seeks to refine the detection performed by the first.

As a result, a segmented image representing at least values of a first value for the pixels occupied by a pancreatic cyst and values of at least a second value for the pixels not occupied by a pancreatic cyst is obtained. For example, the segmented image can represent a binary mask representing with '1' the pixels occupied by a pancreatic cyst an with '0' the pixels not occupied by the pancreatic cyst. Alternatively, the segmented image can represent a mask that presents values of '2' for the pixels occupied by the pancreatic cyst, a value of '1' for the pixels occupied by the pancreas and values of '0' for the rest of pixels.

At step 105, the computer performs a morphological analysis of the pancreatic cyst, particularly using an image processing algorithm, that at least processes the pixels with the first value and at least computes the following features of the pancreatic cyst: a value of eccentricity and of convexity. Finally, at step 106, the pancreatic cyst is classified taking into account the morphological analysis and also demographic data of the patient, including, but not limited to, the gender and age of the patient.

In a particular embodiment, the eccentricity is calculated as follows. From the 3D volume generated by the U-net, which are a set of pixels in the 3D space that shall all together form the detected pancreatic cyst, the slices forming the 3D cyst are identified. For each of this slice, the 2D section of the cyst is calculated and the slice with the biggest section is then selected. Using Image Coordinates System (origin at the top left corner, "x" axis is the horizontal and "y" axis is the vertical) the distance between the point of the section with minimal "x" and maximal "x" is calculated (let's call it "A") , as well as the distance between the point of the section with minimal "y" and maximal "y" (let's call it "B").

Eccentricity is then calculated as: $\dfrac{\sqrt{(\frac{A}{2})^\wedge 2 - (\frac{B}{2})^\wedge 2}}{(\frac{A}{2})}$ . Depending on this value, this part of the decision function will output as more likely one type or another. For example, statistically, pseudocysts are more similar to spheres than other types. This means, when the ratio between axis' is closer to one, the most likely cyst type will be pseudocyst.

Similarly, in a particular embodiment, the convexity can be calculated using the Convex hull technique. This method identifies the 3D points that form the surface of the 3D volume created by the neural network and calculates its equation. Through this approach, first the equation of the smallest convex polygon that includes all pixels equal to two created by the neural network are defined. Secondly, the ratio between the pixels contained in the 3D volume calculated by the neural network and the pixels contained in the surface obtained through Convex hull is calculated. The more similar they are, the close this ratio will be to one, and less lobular shall be then the pancreatic cyst. This impacts on the pancreatic cyst classification since Pseudocyst (e.g.) are less lobular, so if this ratio is closer to one, this part of the decision function will determine that the cyst is a Pseudocyst.

It should be noted that, instead of the above-described methods, other approaches for calculating the eccentricity and convexity values can be alternatively implemented by the image processing algorithm.

In some embodiments, once the eccentricity and convexity values are obtained, the volume of the pancreatic cyst can be also computed. Complementary or alternatively, a relative position value of the detected pancreatic cyst can be also computed. Since the neural network can detect both the pancreas and the pancreatic cyst(s), to determine whether the detected pancreatic cyst(s) is/are located in the head, tail or body of the pancreas, the detected pancreas is divided in 3 thirds in the 3D space. The 3D position of the pancreatic cyst is then checked to see in which third it belongs. This spatial localization of the pancreatic cyst impacts on the cyst classification according to Table 2.

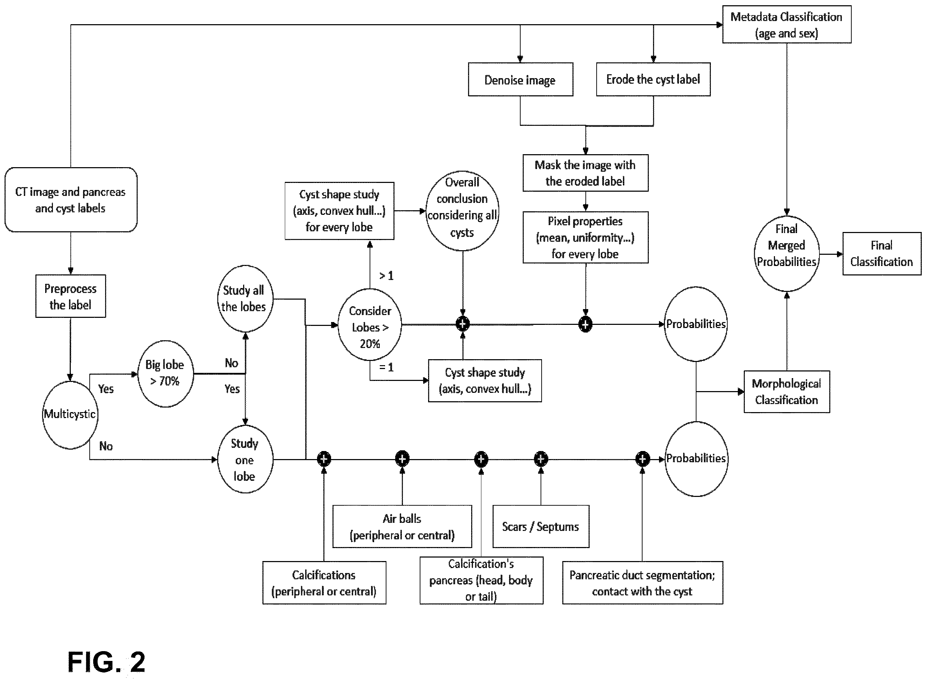

With regard now to FIG. 2, an embodiment of the characterization and classification of a pancreatic cyst is shown. Particularly, the proposed method for classifying pancreatic cysts is based on the following equation 1:

$$C_i = \Pi_i + \theta_i \qquad \text{(Eq. 1)}$$

where Ci represents the class of the i-th cyst; the probability that the cyst belongs to a type according to demographic data (or metadata) is $\Pi_i$ and according to its morphology is $\theta_i$.

In an embodiment, the metadata of the patient that can be taken into account to predict the membership class of the pancreatic cyst are age and sex, according to Table 1:

Table 1.

|  | 0-35 | 40-60 | 60-70 | >80 |
|---|---|---|---|---|
| **WOMAN** | 30% PCYST 20% MCN 20% SCA 0% IPMN | 20% PCYST 50% MCN 20% SCA 10% IPMN | 20% PCYST 20% MCN 50% SCA 10% IPMN | 50% PCYST 10% MCN 10% SCA 30% IPMN |
| **MAN** | 60% PCYST 0% MCN 10% SCA 30% IPMN | 40% PCYST 0% MCN 20% SCA 40% IPMN | 30% PCYST 0% MCN 40% SCA 30% IPMN | 20% PCYST 0% MCN 10% SCA 70% IPMN |

It should be noted that in other embodiments additional metadata can be considered, for example: an ethnic group, a medical history and/or a medication intake of the patient, among others.

In addition, the morphological term ($\theta_i$) can consider the morphology and physical characteristics of the pancreatic cyst. In order to obtain and study the morphology of the pancreatic cyst, it is necessary to extract the labels obtained from the segmented image, which in this particular embodiment is represented by a mask that presents values of '2' for the pixels occupied by the pancreatic cyst, of '1' for the pixels occupied by the pancreas and of '0' for the rest of pixels. Thanks to these results, the pixel information for the pancreatic cyst and the pancreas, respectively, can be obtained.

First, the image is cropped in the area where the pancreas and pancreatic cyst appear to reduce the size of the image that is processed and make the study of the pancreatic cyst faster. Once the image has been cropped and the information from the pixels has been extracted thanks to the labels obtained before (pancreas and cyst), the study of the pancreatic cyst can be started.

First, based on the metadata of the patient, a probability is established for each type of pancreatic cyst. This value will be $\Pi_i$ in Eq. 1.

The morphology (shape and location characteristics) used for the classification to determine the value of $\theta_i$ can be one or more of the following: check if the outline of the pancreatic cyst is flat or lobulated and if it is more circular or ovoid; check cyst pixel properties such as: uniformity, mean, standard deviation; check the location of the pancreatic cyst in relation to the pancreas (head, body or tail); check if the pancreatic cyst is multicystic and, if it is multicystic, check the different cysts and their relevance to the pancreatic cyst as a whole; determine if both the cyst and the pancreas have calcifications and their location; peripheral or central and head, body or tail, respectively; determine if there are air balls in the cyst and their location; peripheral or central; check if there are scars inside the pancreatic cyst; segment the pancreatic duct from the pancreas label and see if the pancreatic cyst is in contact.

According to the available literature on the characterization of pancreatic cysts in medical imaging, in some embodiments, the criteria in Table 2 has been followed to determination of the class of the cyst.

Table 2.

|  | MCN | IPMN | SCA | Pseudocyst |
|---|---|---|---|---|
| **Position in the pancreas** | Body and tail | Head | Any | Any |
| **Morphology** | Berry | Cluster/ Lobular | Berry | Flat |
| **Shape** | Spherical | Oval | Oval | Spherical with well defined margins |
| **Access to pancreatic duct** | NO | YES | NO | NO |

(continued)

|  | MCN | IPMN | SCA | Pseudocyst |
|---|---|---|---|---|
| **Aspect** | Light, viscous, thick walls | Light and viscous | Light | Opaque |

Moreover, depending on the ratio between the pixels within the 3D volume generated by the neural network and the pixels within a calculated convex hull, the following cyst classification can be made.

Table 3.

|  | MCN | IPMN | SCA | Pseudocyst |
|---|---|---|---|---|
| **Ratio detected cyst vs. convex hull** | > 0.5 | < 0.5 | < 0.5 | < 0.5 |
| **Eccentricity** | < 0.5 | < 0.5 | > 0.5 | > 0.5 |
| **Multi-cyst** | NO | YES | NO | NO |

To each characteristic, a probability of each pancreatic cyst can be assigned and, once the respective weights have been assigned for each characteristic, it is compared with the weights of the metadata classification.

In the embodiment of FIG. 2, firstly it is checked if the pancreatic cyst is multicystic. If multicystic, the significance of each pancreatic cyst is assessed. If the largest pancreatic cyst has more than 70% of the total number of pixels, the study is performed considering the largest pancreatic cyst as unique. If not, all pancreatic cysts are considered, but, if a separate lobe is less than 20% of the total number of pixels, it is ignored as it is not considered relevant enough.

For a multiple pancreatic cyst classification, at first it is verified if the mask of the segmented image corresponds to a single region or to multiple unconnected ones. To check if the generated mask corresponds to a unique region, it is checked for each pixel of the mask if at least one of the adjacent pixels has the same value. If none of the adjacent pixels has the same value as the pixel of the mask under study, the pancreatic cyst is considered to be multicystic and each related region of the mask is analyzed independently.

If a classification of multiple pancreatic cysts (more than two cysts <70% and> 20%) is necessary, a study of each pancreatic cyst is performed. First, the convex hull of each pancreatic cyst considered sufficiently important is calculated. Then, the ratio between the number of pixels of the convex hull that contains it and the number of pixels of the lobe is used to study whether the surface is flat or lobed. Subsequently, the proportion between the major axis and the minor axis is used to study whether it is oval or spherical. Next, it will be calculated if there are septums or a central scar from the study of the Hounsfield unit (HU) gradient within the pancreatic cyst. If there is a maximum of HU contained in the center of the pancreatic cyst or in semi-continuous lines, the pancreatic cyst is considered to have septa or a central scar.

For the classification of a single pancreatic cyst, the ratio between its number of pixels and the number of pixels of the respective convex helmet is determined. Subsequently, the standard deviation is also considered, and the respective weights are assigned in each case.

Various aspects of the proposed method, as described herein, may be embodied in programming. Program aspects of the technology may be thought of as "products" or "articles of manufacture" typically in the form of executable code and/or associated data that is carried on or embodied in a type of machine readable medium. Tangible non-transitory "storage" type media include any or all of the memory or other storage for the computers, processors, or the like, or associated modules thereof, such as various semiconductor memories, tape drives, disk drives and the like, which may provide storage at any time for the software programming.

All or portions of the software may, at times, be communicated through a network such as the Internet or various other telecommunication networks. Such communications, for example, may enable loading of the software from one computer or processor into another, for example, from a management server or host computer of a scheduling system into the hardware platform(s) of a computing environment or other system implementing a computing environment or similar functionalities in connection with image processing. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to tangible "storage" media, terms such as computer or machine "readable medium" refer to any medium that participates in providing instructions to a processor for execution.

A machine-readable medium may take many forms, including but not limited to, a tangible storage medium, a carrier wave medium or physical transmission medium. Non-volatile storage media include, for example, optical or magnetic disks, such as any of the storage devices in any computer(s), or the like, which may be used to implement the system or any of its components shown in the drawings. Volatile storage media may include dynamic memory, such as a main memory of such a computer platform. Tangible transmission media may include coaxial cables; copper wire and fiber optics, including the wires that form a bus within a computer system. Carrier-wave transmission media may take the form of electric or electromagnetic signals, or acoustic or light waves such as those generated during radio frequency (RF) and infrared (IR) data communications. Common forms of computer-readable media may include, for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a physical processor for execution.

Those skilled in the art will recognize that the present teachings are amenable to a variety of modifications and/or enhancements. For example, although the implementation of various components described herein may be embodied in a hardware device, it may also be implemented as a software only solution-e.g., an installation on an existing server. In addition, image processing as disclosed herein may be implemented as a firmware, firmware/software combination, firmware/hardware combination, or a hardware/firmware/software combination.

The present disclosure and/or some other examples have been described in the above. According to descriptions above, various alterations may be achieved. The topic of the present disclosure may be achieved in various forms and embodiments, and the present disclosure may be further used in a variety of application programs. All applications, modifications and alterations required to be protected in the claims may be within the protection scope of the present disclosure.

The scope of the present invention is defined in the following set of claims.

**Claims**

1.  A computer implemented method for the automatic classification of pancreatic cysts using computed tomography, CT, images, the method comprising:

    accessing, by a computer, a set of CT images of a patient, each image of the set of CT images representing a different slice;
    performing, by the computer, a filtering operation on the set of CT images, and defining a region of interest, ROI, candidate to contain a pancreatic cyst within the filtered set of CT images;
    performing, by the computer, a segmentation operation of the defined ROI using a neural network, obtaining a segmented image as a result, the segmented image representing values of a first value for the pixels occupied by a pancreatic cyst and values of at least a second value for the pixels not occupied by a pancreatic cyst;
    performing, by the computer, a morphological analysis of the pancreatic cyst using an image processing algorithm that computes a value of eccentricity and of convexity of the pancreatic cyst by processing the pixels with the first value, and
    computes a value of a relative position of the pancreatic cyst by dividing the pixels with the second value in three thirds in a 3D space and by checking a 3D position of the pancreatic cyst within the divided three thirds; and
    classifying, by the computer, the pancreatic cyst based on the morphological analysis performed in the previous step and demographic data of the patient, the demographic data, at least, including the gender and age of the patient.

2.  The method of claim 1, wherein the demographic data of the patient, further includes an ethnic group, a medical history, and/or a medication intake of the patient.

3.  The method of claim 1 or 2, wherein the segmented image represents three different values: the first value for the pixels occupied by the pancreatic cyst, the second value for the pixels occupied by the pancreas and a third value for the rest of the pixels.

4.  The method of any one of the previous claims, wherein the value of eccentricity of the pancreatic cyst is computed by calculating the major and minor axes from a middle section of the pancreatic cyst.

5.  The method of any one of the previous claims, wherein the value of convexity of the pancreatic cyst is computed as a proportion between a volume of the pancreatic cyst and a volume of a respective convex hull.

6. The method of any one of the previous claims, wherein the morphological analysis of the pancreatic cyst further comprises computing one or more of the following features of the pancreatic cysts: if the cyst is multicystic, if it has calcifications and their location, if it has air balls and their location, if there are scars inside the cyst, if the cyst is flat, lobulated, circular or ovoid, an aspect of the cyst and/or if the cyst accesses a pancreatic duct or not.

7. The method of any one of the previous claims, wherein the filtering operation comprises a median filter.

8. The method of claim 7, wherein the median filter comprises a 3 x 3 filter window.

9. The method of any one of the previous claims, wherein the neural network comprises a semantic segmentation algorithm.

10. The method of claim 9, wherein the neural network is based on deep learning, U-Net.

11. The method of any one of the previous claims, wherein the classifying step comprises classifying the pancreatic cyst in four different groups, namely: Intraductal papillary mucinous neoplasia, IPMN; Mucinous cystic neoplasms, MCN; Serous cystadenoma, SCA; and Pseudocysts, PCYST.

12. A computer program product comprising a non-transitory computer-readable storage medium having program instructions embodied therewith, the program instructions executable by at least one hardware processor to:

access a set of CT images of a patient, each image of the set of CT images representing a different slice;
perform a filtering operation on the set of CT images, and define a region of interest, ROI, candidate to contain a pancreatic cyst within the filtered set of CT images;
perform a segmentation operation of the defined ROI using a neural network, obtaining a segmented image as a result, the segmented image representing values of a first value for the pixels occupied by a pancreatic cyst and values of at least a second value for the pixels not occupied by a pancreatic cyst;
perform a morphological analysis of the pancreatic cyst by means of using an image processing algorithm that computes a value of eccentricity and of convexity of the pancreatic cyst by processing the pixels with the first value, and
computes a value of a relative position of the pancreatic cyst by dividing the pixels with the second value in three thirds in a 3D space and by checking a 3D position of the pancreatic cyst within the divided three thirds; and
classify the pancreatic cyst based on the morphological analysis performed in the previous step and demographic data of the patient, the demographic data at least including the gender and age of the patient.

13. The computer program product of claim 12, wherein the segmented image represents three different values: the first value for the pixels occupied by the pancreatic cyst, the second value for the pixels occupied by the pancreas and a third value for the rest of the pixels.

14. The computer program product of claim 12 or 13, wherein the morphological analysis of the pancreatic cyst further comprises computing one or more of the following features of the pancreatic cysts: if the cyst is multicystic, if it has calcifications and their location, if it has air balls and their location, if there are scars inside the cyst, if the cyst is flat, lobulated, circular or ovoid, an aspect of the cyst and/or if the cyst accesses a pancreatic duct or not.

15. The computer program product of any of the previous claims 12 to 14, wherein the classifying step comprises classifying the pancreatic cyst in four different groups, namely: Intraductal papillary mucinous neoplasia, IPMN; Mucinous cystic neoplasms, MCN; Serous cystadenoma, SCA; and Pseudocysts, PCYST.

**Patentansprüche**

1. Computerimplementiertes Verfahren zum automatischen Klassifizieren von Pankreaszysten unter Verwendung von Computertomographie-Bildern, CT-Bildern, wobei das Verfahren Folgendes umfasst:

Zugang zu einem Satz von CT-Bildern eines Patienten durch einen Computer, wobei jedes Bild des Satzes von CT-Bildern eine unterschiedliche Schicht darstellt;
Durchführen einer Filteroperation an dem Satz von CT-Bildern durch den Computer und Definieren eines Bereichs von Interesse, ROI, der eine Pankreaszyste enthalten könnte, innerhalb des gefilterten Satzes von CT-Bildern;

Durchführen einer Segmentierungsoperation des definierten ROI unter Verwendung eines neuronalen Netzwerks durch den Computer, wobei als Ergebnis ein segmentiertes Bild erhalten wird, wobei das segmentierte Bild Werte eines ersten Werts für die durch eine Pankreaszyste belegten Pixel und Werte von mindestens einem zweiten Wert für die nicht durch eine Pankreaszyste belegten Pixel darstellt;

Durchführen einer morphologischen Analyse der Pankreaszyste durch den Computer unter Verwendung eines Bildverarbeitungsalgorithmus, der einen Wert der Exzentrizität und der Konvexität der Pankreaszyste durch Verarbeiten der Pixel mit dem ersten Wert berechnet, und

Berechnen eines Wertes einer relativen Position der Pankreaszyste durch Teilen der Pixel mit dem zweiten Wert in drei Drittel in einem 3D-Raum und durch Überprüfen einer 3D-Position der Pankreaszyste innerhalb der geteilten drei Drittel; und

Klassifizieren der Pankreaszyste durch den Computer, basierend auf der morphologischen Analyse, die in dem vorherigen Schritt durchgeführt wird, und demographischen Daten des Patienten, wobei die demographischen Daten mindestens das Geschlecht und das Alter des Patienten einschließen.

2. Verfahren nach Anspruch 1, wobei die demographischen Daten des Patienten ferner eine ethnische Gruppe, eine Krankengeschichte und/oder eine Medikamenteneinnahme des Patienten einschließen.

3. Verfahren nach Anspruch 1 oder 2, wobei das segmentierte Bild drei unterschiedliche Werte darstellt: den ersten Wert für die durch die Pankreaszyste belegten Pixel, den zweiten Wert für die durch das Pankreas belegten Pixel und einen dritten Wert für die restlichen Pixel.

4. Verfahren nach einem der vorhergehenden Ansprüche, wobei der Wert der Exzentrizität der Pankreaszyste durch Berechnen der Haupt- und Nebenachsen aus einem Mittelabschnitt der Pankreaszyste berechnet wird.

5. Verfahren nach einem der vorhergehenden Ansprüche, wobei der Wert der Konvexität der Pankreaszyste als ein Verhältnis zwischen einem Volumen der Pankreaszyste und einem Volumen einer jeweiligen konvexen Umhüllung berechnet wird.

6. Verfahren nach einem der vorhergehenden Ansprüche, wobei die morphologische Analyse der Pankreaszyste ferner das Berechnen eines oder mehrerer der folgenden Merkmale der Pankreaszysten umfasst: ob die Zyste multizystisch ist, ob sie Verkalkungen aufweist und deren Lage, ob sie Lufteinschlüsse aufweist und deren Lage, ob Narben innerhalb der Zyste vorhanden sind, ob die Zyste flach, gelappt, kreisförmig oder ovoid ist, eines Aspekts der Zyste und/oder ob die Zyste auf einen Pankreasgang Zugang hat oder nicht.

7. Verfahren nach einem der vorhergehenden Ansprüche, wobei die Filteroperation einen Medianfilter umfasst.

8. Verfahren nach Anspruch 7, wobei der Medianfilter ein 3 x 3-Filterfenster umfasst.

9. Verfahren nach einem der vorhergehenden Ansprüche, wobei das neuronale Netzwerk einen semantischen Segmentierungsalgorithmus umfasst.

10. Verfahren nach Anspruch 9, wobei das neuronale Netzwerk auf Deep Learning, U-Net, basiert.

11. Verfahren nach einem der vorhergehenden Ansprüche, wobei der Schritt des Klassifizierens das Klassifizieren der Pankreaszyste in vier unterschiedliche Gruppen umfasst, nämlich: intraduktale papillär-muzinöse Neoplasie, IPMN; muzinöse zystische Neoplasmen, MCN; seröses Zystadenom, SCA; und Pseudozysten, PCYST.

12. Computerprogrammprodukt, umfassend ein nicht-flüchtiges computerlesbares Speichermedium, das darin implementierte Programmanweisungen aufweist, wobei die Programmanweisungen durch mindestens einen Hardware-Prozessor zu Folgendem ausführbar sind:

Zugang zu einem Satz von CT-Bildern eines Patienten, wobei jedes Bild des Satzes von CT-Bildern eine unterschiedliche Schicht darstellt;

Durchführen einer Filteroperation an dem Satz von CT-Bildern und Definieren eines Bereichs von Interesse, ROI, der eine Pankreaszyste enthalten könnte, innerhalb des gefilterten Satzes von CT-Bildern;

Durchführen einer Segmentierungsoperation des definierten ROI unter Verwendung eines neuronalen Netzwerks, wobei als Ergebnis ein segmentiertes Bild erhalten wird, wobei das segmentierte Bild Werte eines ersten Werts für die durch eine Pankreaszyste belegten Pixel und Werte von mindestens einem zweiten Wert für die nicht durch eine Pankreaszyste belegten Pixel darstellt;

Durchführen einer morphologischen Analyse der Pankreaszyste mittels der Verwendung eines Bildverarbeitungsalgorithmus, der einen Wert der Exzentrizität und der Konvexität der Pankreaszyste durch Verarbeiten der Pixel mit dem ersten Wert berechnet, und

Berechnen eines Wertes einer relativen Position der Pankreaszyste durch Teilen der Pixel mit dem zweiten Wert in drei Drittel in einem 3D-Raum und durch Überprüfen einer 3D-Position der Pankreaszyste innerhalb der geteilten drei Drittel; und

Klassifizieren der Pankreaszyste, basierend auf der morphologischen Analyse, die in dem vorherigen Schritt durchgeführt wird, und demographischen Daten des Patienten, wobei die demographischen Daten mindestens das Geschlecht und das Alter des Patienten einschließen.

13. Computerprogrammprodukt nach Anspruch 12, wobei das segmentierte Bild drei unterschiedliche Werte darstellt: den ersten Wert für die durch die Pankreaszyste belegten Pixel, den zweiten Wert für die durch das Pankreas belegten Pixel und einen dritten Wert für die restlichen Pixel.

14. Computerprogrammprodukt nach Anspruch 12 oder 13, wobei die morphologische Analyse der Pankreaszyste ferner das Berechnen eines oder mehrerer der folgenden Merkmale der Pankreaszysten umfasst: ob die Zyste multizystisch ist, ob sie Verkalkungen aufweist und deren Lage, ob sie Lufteinschlüsse aufweist und deren Lage, ob Narben innerhalb der Zyste vorhanden sind, ob die Zyste flach, gelappt, kreisförmig oder ovoid ist, eines Aspekts der Zyste und/oder ob die Zyste auf einen Pankreasgang Zugang hat oder nicht.

15. Computerprogrammprodukt nach einem der vorhergehenden Ansprüche 12 bis 14, wobei der Schritt des Klassifizierens das Klassifizieren der Pankreaszyste in vier unterschiedliche Gruppen umfasst, nämlich: intraduktale papillär-muzinöse Neoplasie, IPMN; muzinöse zystische Neoplasmen, MCN; seröses Zystadenom, SCA; und Pseudozysten, PCYST.

## Revendications

1. Procédé mis en oeuvre par ordinateur pour la classification automatique des kystes pancréatiques en utilisant des images de tomodensitométrie, TDM, le procédé comprenant :

l'accès, par un ordinateur, à un ensemble d'images de TDM d'un patient, chaque image de l'ensemble d'images de TDM représentant une tranche différente ;

la réalisation, par l'ordinateur, d'une opération de filtrage sur l'ensemble d'images de TDM, et la définition d'une région d'intérêt, ROI, candidate pour contenir un kyste pancréatique au sein de l'ensemble d'images de TDM filtrées ;

la réalisation, par l'ordinateur, d'une opération de segmentation de la ROI définie en utilisant un réseau neuronal, en obtenant une image segmentée en résultat, l'image segmentée représentant des valeurs d'une première valeur pour les pixels occupés par un kyste pancréatique et des valeurs d'au moins une deuxième valeur pour les pixels non occupés par un kyste pancréatique ;

la réalisation, par l'ordinateur, d'un analyse morphologique du kyste pancréatique en utilisant un algorithme de traitement d'images qui calcule une valeur d'excentricité et de convexité du kyste pancréatique en traitant les pixels avec la première valeur, et

calcule une valeur d'une position relative du kyste pancréatique en divisant les pixels avec la deuxième valeur en trois tiers dans un espace en 3D et en vérifiant une position en 3D du kyste pancréatique au sein des trois tiers divisés ; et

la classification, par l'ordinateur, du kyste pancréatique sur la base de l'analyse morphologique réalisé dans l'étape précédente et de données démographiques du patient, les données démographiques comportant, au moins, le sexe et l'âge du patient.

2. Procédé selon la revendication 1, dans lequel les données démographiques du patient comportent en outre un groupe ethnique, des antécédents médicaux et/ou une prise de médicaments du patient.

3. Procédé selon les revendications 1 ou 2, dans lequel l'image segmentée représente trois valeurs différentes : la première valeur pour les pixels occupés par le kyste pancréatique, la deuxième valeur pour les pixels occupés par le pancréas et une troisième valeur pour le reste des pixels.

4.  Procédé selon l'une quelconque des revendications précédentes, dans lequel la valeur d'excentricité du kyste pancréatique est calculée en calculant les axes majeur et mineur à partir d'une section centrale du kyste pancréatique.

5.  Procédé selon l'une quelconque des revendications précédentes, dans lequel la valeur de convexité du kyste pancréatique est calculée comme une proportion entre un volume du kyste pancréatique et un volume d'une coque convexe respective.

6.  Procédé selon l'une quelconque des revendications précédentes, dans lequel l'analyse morphologique du kyste pancréatique comprend en outre le calcul d'une ou plusieurs des caractéristiques suivantes des kystes pancréatiques : si le kyste est multikystique, s'il a des calcifications et leurs emplacement, s'il a des boules d'air et leur emplacement, s'il y a des cicatrices à l'intérieur du kyste, si le kyste est plat, lobé, circulaire ou ovoïde, un aspect du kyste et/ou si le kyste accède à un conduit pancréatique ou non.

7.  Procédé selon l'une quelconque des revendications précédentes, dans lequel l'opération de filtrage comprend un filtre médian.

8.  Procédé selon la revendication 7, dans lequel le filtre médian comprend une fenêtre de filtrage de 3 x 3.

9.  Procédé selon l'une quelconque des revendications précédentes, dans lequel le réseau neuronal comprend un algorithme de segmentation sémantique.

10. Procédé selon la revendication 9, dans lequel le réseau neuronal est basé sur l'apprentissage profond, U-Net.

11. Procédé selon l'une quelconque des revendications précédentes, dans lequel l'étape de classification comprend la classification du kyste pancréatique en quatre groupes différents, à savoir : une néoplasie mucineuse paipllaire intracanalaire, IPMN ; des néoplasmes kystiques mucineux, MCN ; un cystadénome séreux, SCA ; et des pseudokystes, PCYST.

12. Produit de programme informatique comprenant un support de stockage lisible par ordinateur non transitoire ayant des instructions de programme incorporés dans celui-ci, les instructions de programme étant exécutables par au moins un processeur matériel pour :

    accéder à un ensemble d'images de TDM d'un patient, chaque image de l'ensemble d'images de TDM représentant une tranche différente ;
    réaliser une opération de filtrage sur l'ensemble d'images de TDM, et définir une région d'intérêt, ROI, candidate pour contenir un kyste pancréatique au sein de l'ensemble d'images de TDM filtrées ;
    réaliser une opération de segmentation de la ROI définie en utilisant un réseau neuronal, en obtenant une image segmentée en résultat, l'image segmentée représentant des valeurs d'une première valeur pour les pixels occupés par un kyste pancréatique et des valeurs d'au moins une deuxième valeur pour les pixels non occupés par un kyste pancréatique ;
    réaliser un analyse morphologique du kyste pancréatique par le biais de l'utilisation d'un algorithme de traitement d'images qui calcule une valeur d'excentricité et de convexité du kyste pancréatique en traitant les pixels avec la première valeur, et
    calcule une valeur d'une position relative du kyste pancréatique en divisant les pixels avec la deuxième valeur en trois tiers dans un espace en 3D et en vérifiant une position en 3D du kyste pancréatique au sein des trois tiers divisés ; et
    classifier le kyste pancréatique sur la base de l'analyse morphologique réalisé dans l'étape précédente et de données démographiques du patient, les données démographiques comportant, au moins, le sexe et l'âge du patient.

13. Produit de programme informatique selon la revendication 12, dans lequel l'image segmentée représente trois valeurs différentes : la première valeur pour les pixels occupés par le kyste pancréatique, la deuxième valeur pour les pixels occupés par le pancréas et une troisième valeur pour le reste des pixels.

14. Produit de programme informatique selon les revendications 12 ou 13, dans lequel l'analyse morphologique du kyste pancréatique comprend en outre le calcul d'une ou plusieurs des caractéristiques suivantes des kystes pancréatiques : si le kyste est multikystique, s'il a des calcifications et leur emplacement, s'il a des boules d'air et leur emplacement, s'il y a des cicatrices à l'intérieur du kyste, si le kyste est plat, lobé, circulaire ou ovoïde, un aspect du kyste et/ou si le kyste accède à un conduit pancréatique ou non.

15. Produit de programme informatique selon l'une quelconque des revendications précédentes 12 à 14, dans lequel l'étape de classification comprend la classification du kyste pancréatique en quatre groupes différentes, à savoir : une néoplasie mucineuse paipllaire intracanalaire, IPMN ; des néoplasmes kystiques mucineux, MCN ; un cystadénome séreux, SCA ; et des pseudokystes, PCYST.

Access images of a patient 101

Perform filtering operation on the set of images 102

Define a ROI to contain a pancreatic cyst within the filtered images 103

Perform segmentation operation of the defined ROI using neural network 104

Perform morphological analysis of the pancreatic cyst using image processing algorithm 105

Classify the pancreatic cyst using the morphological analysis and demographic data 106

# FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3646240 A1
- WO 2021096991 A1
- US 20210012505 A1
- EP 1421544 A1
- WO 2020240455 A1
- CN 108898152 B

**Non-patent literature cited in the description**

- **DMITRIEV et al.** *Classification of Pancreatic Cysts in Computed Tomography Images Using a Random Forest and Convolutional Neural Network Ensemble*
- **ATTIYEH et al.** *CT radiomics associations with genotype and stromal content in pancreatic ductal adenocarcinoma*